## Europäisches Patentamt
## European Patent Office
## Office européen des brevets

(19)

(11) Numéro de publication : **0 059 668**
**B1**

(12)

# FASCICULE DE BREVET EUROPÉEN

(45) Date de publication du fascicule du brevet :
30.10.85

(21) Numéro de dépôt : **82400338.8**

(22) Date de dépôt : **26.02.82**

(51) Int. Cl.⁴ : **H 05 H 6/00, G 21 K 1/00, A 61 N 5/10**

(54) **Générateur de neutrons de grande énergie.**

(30) Priorité : **02.03.81 FR 8104109**

(43) Date de publication de la demande :
**08.09.82 Bulletin 82/36**

(45) Mention de la délivrance du brevet :
**30.10.85 Bulletin 85/44**

(84) Etats contractants désignés :
**BE CH DE GB IT LI NL SE**

(56) Documents cités :
**FR-A- 2 386 230**
**US-A- 2 816 242**
**US-A- 3 860 827**
**US-A- 4 236 965**
**NUCLEAR INSTRUMENTS AND METHODS, vol.89, 1970, North-Holland Publishing Co., Amsterdam (NL), J.A. JUNGERMAN et al.:"A Medium-Energy Neutron facility", pages 167-172**

(73) Titulaire : **COMMISSARIAT A L'ENERGIE ATOMIQUE Etablissement de Caractère Scientifique Technique et Industriel**
**31/33, rue de la Fédération**
**F-75015 Paris (FR)**

**CENTRE ANTOINE LACASSAGNE**
**36, Avenue de la Voie Romaine**
**F-06054 Nice (FR)**

(72) Inventeur : **Barjon, Robert de l'Université Scientifique et**
**Médicale de Grenoble 4, Boulevard Joseph Vallier**
**F-38000 Grenoble (FR)**
Inventeur : **Breynat, Geneviève**
**A l'Energie Atomique Cidex 7 Bernin**
**F-38190 Brignoud (FR)**
Inventeur : **Mandrillon, Pierre**
**51, route de la Faucille**
**F-01630 Saint Genis Pouilly (FR)**

(74) Mandataire : **Mongrédien, André et al**
**c/o BREVATOME 25, rue de Ponthieu**
**F-75008 Paris (FR)**

Jouve, 18, rue St-Denis, 75001 Paris, France

## Description

La présente invention concerne un générateur de neutrons de grande énergie. Elle s'applique notamment à la neutronothérapie.

On sait que les générateurs de neutrons actuellement utilisés en neutronothérapie comprennent :

soit, ainsi que décrit dans le document FR-A-2 386 230, un accélérateur de particules (cyclotron, linéaire à protons ou tandem), bombardant avec ces particules (protons ou noyaux de deutérium appelés « deutérons »), d'énergie comprise entre 15 et 60 MeV, une cible de béryllium, de lithium ou de carbone 13 ;

soit un accélérateur de particules, bombardant avec des deutérons de 150 à 500 keV une cible tritiée, ou bombardant, avec un mélange de deutérons et de noyaux de tritium (appelés « tritons ») de 150 à 500 keV, une cible de métal hydrurable (« autocible », cette cible se régénérant), de façon à produire des neutrons d'énergie égale à 15 MeV, qui sont déjà très efficaces en neutronothérapie.

Or, de tels générateurs, notamment à cause de l'épaisseur et de la nature des cibles qu'ils comportent, ne permettent pas d'obtenir des faisceaux de neutrons de grande énergie aussi intenses et aussi peu contaminés par des neutrons d'énergie inférieure à 15 MeV qu'il serait souhaitable.

La présente invention a pour but de remédier à ces inconvénients.

Elle a pour objet un générateur de neutrons rapides peu contaminés en neutrons de faible énergie, du genre de ceux qui comprennent :

une source de particules chargées d'énergie au moins égale à 15 MeV,

une cible faite d'un matériau apte à émettre des neutrons sous l'impact de ces particules chargées, et

des moyens de refroidissement de cette cible, caractérisé en ce que la cible est constituée par au moins deux éléments séparés l'un de l'autre et faits de deutérure de lithium, l'épaisseur de chacun de ces éléments étant adaptée pour que la cible produise principalement, en avant, des neutrons d'énergie au moins égale à 15 MeV, et en ce que les moyens de refroidissement de la cible consistent en des moyens de circulation permettant de faire circuler entre lesdits éléments un gaz ne réagissant pas chimiquement avec le deutérure de lithium.

Lesdits éléments peuvent être de formes géométriques variées.

Par « neutrons produits en avant », on entend des neutrons émis par la cible dans des directions voisines de celle des particules chargées incidentes.

La source de ces particules chargées est par ailleurs prévue pour émettre ces particules avec une énergie déterminée en fonction de celle des neutrons à produire en avant. (Des valeurs de l'énergie à communiquer à ces particules chargées seront données à titre d'exemple par la suite).

Quant au choix de la cible, les particules chargées incidentes étant par exemple des protons, plus les atomes constituant cette cible sont légers, plus la quantité de neutrons émis par interaction directe — interaction prédominante pour les énergies considérées — est importante et plus la quantité de neutrons d'énergie inférieure, résultants de chocs secondaires, est faible.

Parmi les atomes dits légers, (c'est-à-dire de numéros atomiques inférieurs à celui du carbone), il est bien entendu préférable de choisir ceux qui contiennent le plus de neutrons pour constituer la cible.

En éliminant de la liste des matériaux que l'on pourrait utiliser le deutérium et des composés normalement gazeux et très légers comme $CD_4$, pour des questions de coût d'installation et d'exploitation (nécessité de liquéfaction pour avoir une densité suffisante), le tritium, à cause de son prix, de sa radioactivité et de difficultés de mise en œuvre, et l'hélium, pour la valeur élevée de l'énergie absorbée par une réaction (p, n) avec cet élément, on est conduit à réaliser, pour le générateur de neutrons objet de l'invention, une cible en deutérure de lithium LiD, (selon une caractéristique préférée de l'invention, en deutérure de lithium 7 ou en deutérure de lithium naturel) car, pour un même nombre de neutrons-cibles et de protons incidents et pour une même perte d'énergie de ces derniers, la réaction (p, n) a une probabilité plus de deux fois plus grande avec la molécule $^7LiD$ (mais seulement environ une fois et demie plus grande avec l'atome de lithium 7) qu'avec une molécule de béryllium, d'eau lourde ou de paraffine lourde.

On obtient donc avec le générateur selon l'invention, muni d'une cible en LiD, et de préférence en $^7LiD$, une quantité de neutrons supérieure à celle obtenue avec les générateurs de l'art antérieur qui utilisaient des cibles en béryllium.

Lesdites particules chargées peuvent être par exemple des deutérons, mais ce sont de préférence des protons. En effet, pour produire, en avant, des neutrons d'énergie E au moins égale à 15 MeV, on peut utiliser, l'interaction directe étant favorisée à de telles énergies, un courant d'intensité I de protons d'énergie E ou un courant d'intensité 1/2 de deutérons d'énergie 2E, pour une même puissance du faisceau incident de protons ou de deutérons. Or, le coût d'un cyclotron, meilleure source de protons ou de deutérons aux énergies envisagées, dépend beaucoup plus de l'énergie à communiquer à ces particules (du fait que cette énergie est une fonction croissante du champ magnétique et des dimensions du cyclotron) que de l'intensité du courant à produire desdites particules. Selon une caractéristique préférée de l'invention, ces dernières sont donc des protons, de façon à réduire le coût du générateur objet de l'invention.

Le matériau (LiD) constituant la cible utilisée dans l'invention a une épaisseur limitée par rapport à celle des cibles de l'art antérieur et déterminée pour ne produire, en avant, que très peu de neutrons d'énergie inférieure à 15 MeV sous l'impact des particules chargées issues de la source. Pour ce faire, il suffit de donner audit matériau une épaisseur telle que les particules chargées incidentes n'ayant pas réagi avec lui aient encore à la sortie de la cible une énergie au moins égale à 15 MeV lorsque ces particules chargées sont des protons et au moins égale à 30 MeV lorsqu'il s'agit de deutérons.

Par exemple, avec des protons incidents de 50 MeV, l'épaisseur de LiD est calculée pour que ceux de ces protons qui n'ont pas été arrêtés dans la cible aient encore au moins 15 MeV à la sortie de cette cible qui est alors dite « semi-épaisse ».

En réduisant ainsi l'épaisseur de la cible par rapport à celles de l'art antérieur, on diminue également d'environ 30 % son échauffement.

De préférence, la cible a une épaisseur ajustée en fonction de l'énergie moyenne des particules chargées que la source est apte à émettre et de l'énergie des neutrons à produire, et comporte environ M éléments, consistant en des pastilles, chaque pastille ayant une épaisseur différente, calculée pour que les particules chargées issues de la source y perdent la fraction 1/2 M de leur énergie, ces pastilles étant espacées et rangées par épaisseurs décroissantes à partir de la source de particules chargées.

Il peut être important d'arrêter les particules chargées incidentes, partiellement ralenties, issues de la cible. C'est notamment le cas en neutronothérapie où l'on ne veut pas que ces particules puissent atteindre un malade en cours de traitement. Ainsi, dans les générateurs connus, le refroidissement de la cible se fait-il généralement par circulation d'eau, à l'arrière de cette cible, ce qui permet d'absorber une partie des particules incidentes telles que des protons n'ayant pas réagi avec la cible et ralenties par elle, mais présente en revanche l'inconvénient de dégrader partiellement le spectre en énergie des neutrons créés par la cible.

Pour remédier à cet inconvénient, les moyens de refroidissement de la cible du générateur objet de l'invention consistent donc en des moyens de circulation d'un gaz ne réagissant pas chimiquement avec ledit matériau dont est faite la cible. Ce gaz peut être de l'hélium dont la section efficace de diffusion est particulièrement faible pour les neutrons de grande énergie.

Selon une caractéristique particulière, le générateur de neutrons objet de l'invention comprend en outre des moyens d'élimination de particules parasites sortant en avant de la cible, ces particules parasites étant entre autres les particules chargées n'ayant pas réagi avec la cible et les neutrons issus de cette dernière et d'énergie inférieure à 15 MeV.

Selon une autre caractéristique préférée, les moyens d'élimination de particules parasites comprennent des moyens de déviation des particules chargées non absorbées par la cible pour écarter celles-ci des neutrons produits en avant, de façon que ces derniers soient principalement des neutrons d'énergie au moins égale à 15 MeV.

Selon une autre caractéristique particulière, le générateur de neutrons objet de l'invention comprend en outre un collimateur destiné à définir un champ d'irradiation.

Lesdits moyens de déviation peuvent être prévus pour envoyer lesdites particules chargées non absorbées par la cible vers une paroi du collimateur. Un bloc d'arrêt, muni d'un système réfrigérant et de préférence essentiellement constitué de carbone ou d'hélium, peut alors être placé dans ladite paroi du collimateur, pour arrêter lesdites particules chargées non absorbées par la cible.

Selon une autre caractéristique particulière, lesdits moyens de déviation sont aptes à engendrer un champ magnétique pour provoquer ladite déviation. Ils peuvent consister en un aimant permanent. Ce dernier peut être au moins en partie intégré aux parois du collimateur, celles-ci étant aptes à constituer un circuit magnétique.

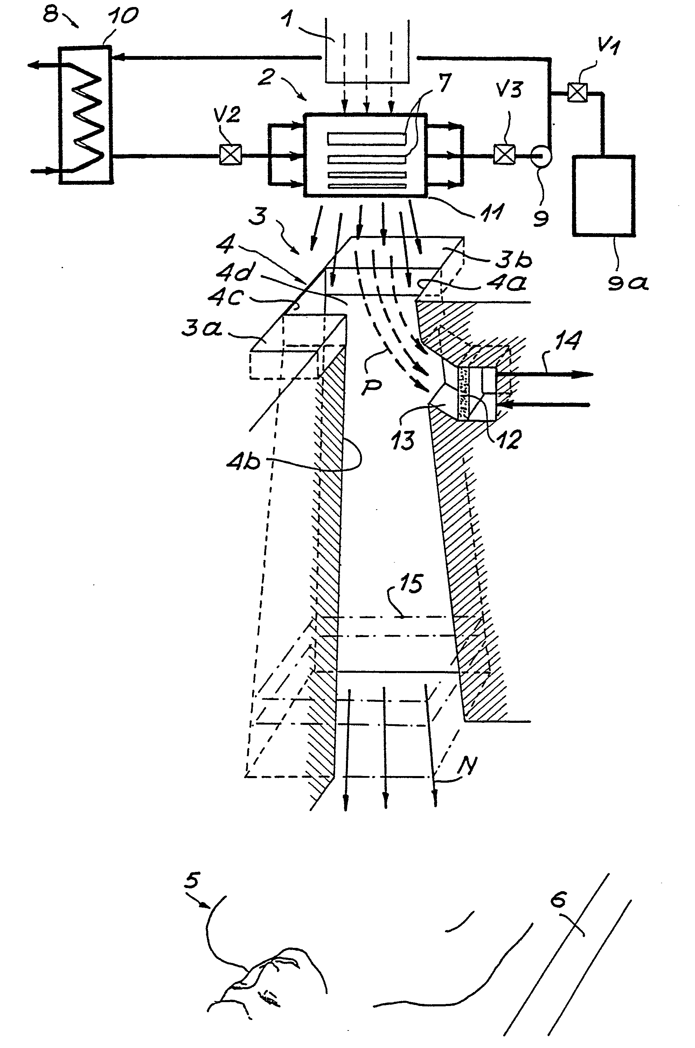

D'autres caractéristiques et avantages du générateur objet de l'invention apparaîtront mieux à la lecture de la description qui suit d'un exemple de réalisation donné à titre indicatif et non limitatif en référence au dessin annexé.

Sur ce dessin, on a représenté schématiquement un mode de réalisation particulier du générateur de neutrons de grande énergie selon l'invention, appliqué à la neutronothérapie. Il comporte essentiellement une source 1 de protons d'énergie au moins égale à 15 MeV, envoyant ceux-ci sur une cible 2 engendrant alors des neutrons N de grande énergie (au moins égale à 15 MeV). Un aimant permanent 3, placé à la suite de la cible 2, dévie les protons P ayant traversé la cible 2 sans réagir avec elle pour créer des neutrons. Un collimateur 4 permet de définir un champ d'irradiation pour un malade 5 placé sur une table 6. Pour pouvoir irradier le malade 5 à l'endroit voulu, le générateur objet de l'invention et la table 6 sont déplaçables à l'aide de moyens non représentés sur le dessin, connus dans l'état de la technique et ne faisant pas partie de l'invention.

La source 1 est un accélérateur de particules, tel qu'un cyclotron, qui fournit des protons dont l'énergie est par exemple voisine de 50 MeV. (Dans le cas d'un cyclotron, les protons peuvent être envoyés sur la cible 2 à l'aide de moyens de déflexion magnétiques non représentés sur le dessin et connus dans l'état de la technique).

La cible 2 est une cible « semi-épaisse » : le matériau dont elle est faite est d'épaisseur telle que les protons incidents de 50 MeV issus de la source 1 et non arrêtés par la cible aient par exemple perdu dans cette dernière au plus la moitié de leur énergie ; ainsi, juste avant de sortir de la cible 2, les protons qui n'avaient pas encore été arrêtés par elle peuvent-ils l'être et produire alors par choc direct des neutrons d'énergie voisine de 25 MeV. Tous les neutrons créés, en avant, dans la cible ont donc au moins une énergie voisine de 25 MeV, énergie bien supérieure à 15 MeV. On élimine ainsi du faisceau de neutrons rapides N atteignant le malade 5 une partie des particules parasites qu'il contenait généralement dans les générateurs de l'art antérieur, partie constituée de neutrons de faible énergie (inférieure à 15 MeV).

Le matériau constituant la cible 2 est du deutérure de lithium 7 ou naturel. Outre les avantages mentionnés plus haut, LiD présente l'intérêt d'avoir un point de fusion élevé (supérieur à 600 °C).

Afin d'être convenablement refroidie d'une façon qui sera expliquée par la suite, la cible 2 comporte plusieurs pastilles 7 en LiD, séparées les unes des autres. Il y a par exemple une dizaine de pastilles, chacune d'elles ayant une épaisseur différente, calculée pour que les protons issus de la source 1 y perdent le vingtième de leur énergie. Ces pastilles 7 sont rangées par épaisseurs décroissantes à partir de la source 1 de protons. Par ailleurs, la somme des épaisseurs des pastilles 7 est ajustée en fonction de l'énergie moyenne (50 MeV) des protons que la source 1 peut émettre et de l'énergie des neutrons de grande énergie à produire. Par interaction avec la cible 2, les protons issus de la source 1 peuvent donc produire des neutrons dont l'énergie est au moins de l'ordre de 25 MeV car les protons perdent au plus une énergie voisine de $10 \times 1/20 \times 50 = 25$ MeV dans la cible 2.

Le deutérure de lithium se présentant normalement sous forme cristalline en grains dont la grosseur varie suivant le mode de fabrication, on le concasse et on le pulvérise, puis on en fait des lingots dans une presse à une température inférieure à 600 °C pour que LiD ne se décompose pas, lingots que l'on peut découper pour obtenir lesdites pastilles.

Des moyens 8 de refroidissement de la cible 2 permettent de faire circuler, entre les pastilles 7, un gaz ne réagissant pas chimiquement avec LiD. Ce gaz peut être de l'azote ou, de préférence, de l'hélium (néanmoins plus coûteux que l'azote) qui ne perturbe que très peu l'énergie des neutrons produits dans la cible 2 (et qui est par ailleurs lui-même capable d'en produire). Le gaz est recyclé à l'aide d'un compresseur 9, après refroidissement éventuel dans un échangeur de chaleur 10. Les moyens 8 de refroidissement comportent également une cuve de stockage 9a isolable par une vanne $V_1$. La cible 2, contenue dans un récipient étanche 11 isolable du reste desdits moyens 8 de refroidissement par deux vannes $V_2$ et $V_3$, peut se démonter et se changer facilement. De plus, des canalisations d'hélium sous pression (2,5 bars au maximum) sont d'un emploi plus facile avec un générateur mobile que des canalisations d'eau telles que celles employées dans les générateurs de neutrons de l'art antérieur. Par ailleurs, le fait de ne pas utiliser un refroidissement par circulation d'eau diminue beaucoup les besoins de protection ; (avec des flux élevés de neutrons, la radioactivité induite dans l'eau est importante et la dissociation de l'eau en hydrogène et oxygène par radiolyse l'est également).

Les protons P incidents qui, n'ayant pas participé à la réaction nucléaire « neutronigène », c'est-à-dire productrice de neutrons, se sont ralentis dans la cible 2 sans s'y arrêter, ont, comme on l'a vu, une énergie bien supérieure à 15 MeV et donc un parcours d'au moins deux mètres dans l'air. Or, dans l'application neutronothérapique du générateur de neutrons objet de l'invention, la cible 2 est à une vingtaine de centimètres du collimateur 4 qui présente une épaisseur d'au plus 120 centimètres. Ainsi, le malade 5 se trouve-t-il au maximum à 140 centimètres de la cible 2 et serait donc atteint par les protons P ralentis mais non arrêtés par la cible 2 si ces protons P n'étaient pas empêchés d'atteindre le malade 5.

Pour arrêter ces protons P, on pourrait utiliser une lame épaisse d'un matériau le plus lourd possible comme le tungstène (pour limiter le plus possible le nombre de neutrons de faible énergie créés par interaction des protons P avec la lame) mais cette lame dégraderait le spectre des neutrons N utiles (de grande énergie) lorsqu'ils la traverseraient. Pour éviter cet inconvénient, il est préférable d'écarter les protons P non absorbés par la cible 2, mais ralentis par celle-ci, du faisceau des neutrons N de grande énergie, à l'aide d'un champ magnétique et de les arrêter dans un piège, comme on le verra plus loin, ce qui supprime également les neutrons secondaires (de faible énergie) et les photons γ que ces protons P sont susceptibles d'engendrer. (Bien entendu, on pourrait utiliser des moyens de déflexion électrostatiques pour ces protons P).

Ledit champ magnétique est créé par l'aimant permanent 3. Un électro-aimant serait moins onéreux que cet aimant permanent, mais ce dernier est préférable du fait de sa grande fiabilité (c'est un élément passif et pratiquement inusable) et de la facilité de sa mise en œuvre.

Le collimateur 4, par exemple en alliage fer-cadmium, comporte quatre parois 4a, 4b, 4c et 4d prévues pour délimiter un trou par exemple de section carrée et allant en s'évasant de son extrémité en regard de la cible 2 à son extrémité en regard du malade 5 pour définir un champ d'irradiation de ce dernier par les neutrons N de grande énergie. Pour une meilleure compréhension du dessin, ce collimateur 4 est représenté en coupe partielle sur ce dessin.

L'aimant permanent 3 comporte deux parties actives 3a et 3b se faisant face, pour former les pôles Nord et Sud de l'aimant. Ces parties 3a et 3b sont placées au voisinage de la cible 2 tout en étant intégrées aux parois du collimateur 4. Cette disposition de l'aimant 3 s'explique par la double nécessité de placer cet aimant près de la cible 2 pour éviter qu'il ait un entrefer trop important, étant donné la grande divergence du faisceau des protons P sortant de la cible 2 (divergence due aux diffusions multiples des protons sur les noyaux de cette cible) et d'avoir une induction magnétique qui exerce son action sur une longueur suffisamment importante afin d'éliminer le plus rapidement possible les protons P du faisceau des neutrons N thérapeutiques.

Les parois du collimateur 4 permettent de fermer le circuit magnétique de l'aimant permanent 3 en remplaçant, au voisinage de ce dernier, l'alliage de fer-cadmium constituant lesdites parois par du fer.

Les protons P non absorbés par la cible 2 sont donc déviés vers l'une 4a de ces parois. Un bloc d'arrêt 12, disposé dans une cavité 13 pratiquée dans la paroi 4a, est prévu pour arrêter ces protons P. Ce bloc d'arrêt 12, du type cage de Faraday, et essentiellement constitué de carbone afin de minimiser la production de photons γ, est muni d'un système réfrigérant 14 destiné à éliminer la puissance dégagée lors de l'impact des protons P sur ce bloc 12, puissance qui s'élève à environ 750 W pour une intensité de 30 μA de protons P de 25 MeV.

Selon l'art antérieur, certains générateurs connus de neutrons de grande énergie comprennent des filtres égalisateurs dont le rôle est de compenser une anisotropie de la fluence neutronique à la sortie du collimateur, l'irradiation du malade devant être uniforme. Cette anisotropie est due aux cibles épaisses employées qui diffusent les neutrons en changeant leur énergie et leur direction, la fluence neutronique étant alors plus importante au bord du faisceau de neutrons produits qu'en son centre. Ces filtres égalisateurs absorbent également les neutrons de faible énergie mais réduisent d'une façon notable l'énergie moyenne de l'ensemble des neutrons créés. Ils ont une épaisseur importante et sont faits de matériaux fortement hydrogénés tels que le polyéthylène ou la paraffine.

L'aimant permanent 3, en déviant les protons P non absorbés, générateurs de neutrons de faible énergie, permet de diminuer le nombre et l'épaisseur de ces filtres égalisateurs. Par ailleurs, dans le générateur selon l'invention, l'emploi de la cible 2 « semi-épaisse » permet de réduire considérablement non seulement la quantité de neutrons de faible énergie présents dans le faisceau de neutrons N thérapeutiques comme on l'a vu précédemment, mais encore l'anisotropie de la fluence neutronique, du fait de l'épaisseur réduite de la cible 2 par rapport à l'art antérieur. Cela permet donc de supprimer en grande partie les filtres égalisateurs. Eventuellement, on peut placer un tel filtre 15 à la sortie du collimateur 4, du côté du malade 5.

Le générateur de neutrons de grande énergie décrit précédemment comportant une cible 2 « semi-épaisse » en deutérure de lithium 7 ou naturel, un aimant permanent 3 de déviation des protons P non absorbés par la cible 2, et éventuellement un filtre égalisateur 15 très réduit, est capable de produire un flux de neutrons N de grande énergie d'une part élevé et d'autre part très faiblement contaminé par des particules parasites (protons, neutrons de faible énergie, photons γ). Cela modifie le rendement d'irradiation en profondeur et conditionne la dose transmise à la zone irradiée du malade dans un sens favorable aux exigences médicales. On peut ainsi réduire d'un facteur de l'ordre de trois la durée d'irradiation par rapport aux générateurs de l'art antérieur, pour un même courant de protons sur la cible.

Dans ces conditions, le temps de mise en place d'un malade devant le faisceau de neutrons est bien plus important que le temps d'irradiation de ce malade. Il est alors possible et économiquement intéressant de disposer d'une salle d'irradiation supplémentaire par rapport aux cas actuellement rencontrés dans lesquels on ne prévoit qu'une salle d'irradiation (au mieux deux) pour un accélérateur de particules. Dans ce cas, et pour abaisser le coût du générateur, les mêmes moyens 8 de refroidissement peuvent être utilisés pour chacune des cibles 2 correspondant aux différentes salles d'irradiation.

Le générateur de neutrons de grande énergie objet de l'invention pourrait être également utilisé pour la production de grands flux de neutrons rapides (environ $10^{13}$ neutrons/cm$^2$/s), d'environ 15 MeV, destinés à l'étude des matériaux de structure des réacteurs de fusion nucléaire (étude sur éprouvettes des dommages causés par le ralentissement des neutrons créés dans les réactions deutéron-triton et deutéron-deutéron dans ces réacteurs).

**Revendications**

1. Générateur de neutrons rapides peu contaminés en neutrons de faible énergie, du genre de ceux qui comprennent :
   une source (1) de particules chargées d'énergie au moins égale à 15 MeV,
   une cible (2) faite d'un matériau apte à émettre des neutrons sous l'impact de ces particules chargées, et
   des moyens (8) de refroidissement de cette cible (2), caractérisé en ce que la cible (2) est constituée par au moins deux éléments (7) séparés l'un de l'autre et faits de deutérure de lithium, l'épaisseur de chacun de ces éléments étant adaptée pour que la cible produise principalement, en avant, des neutrons d'énergie au moins égale à 15 MeV, et en ce que les moyens (8) de refroidissement de la cible (2) consistent en des moyens de circulation permettant de faire circuler entre lesdits éléments un gaz ne réagissant pas chimiquement avec le deutérure de lithium.

2. Générateur de neutrons selon la revendication 1, caractérisé en ce que les particules chargées sont des protons.

3. Générateur de neutrons selon l'une quelconque des revendications 1 et 2, caractérisé en ce que les éléments sont en deutérure de lithium 7 ou en deutérure de lithium naturel.

4. Générateur de neutrons selon l'une quelconque des revendications 1 à 3, caractérisé en ce que la cible (2) a une épaisseur ajustée en fonction de l'énergie moyenne des particules chargées que la source (1) est apte à émettre et de l'énergie des neutrons à produire, et comporte environ M éléments (7), consistant en des pastilles, chaque pastille ayant une épaisseur différente, calculée pour que les particules chargées issues de la source (1) y perdent la fraction 1/2 M de leur énergie, ces pastilles (7) étant espacées et rangées par épaisseurs décroissantes à partir de la source (1) de particules chargées.

5. Générateur de neutrons selon l'une quelconque des revendications 1 à 4, caractérisé en ce que ledit gaz est de l'hélium.

6. Générateur de neutrons selon l'une quelconque des revendications 1 à 5, caractérisé en ce qu'il comprend en outre des moyens (3, 4) d'élimination de particules parasites sortant en avant de la cible (2).

7. Générateur de neutrons selon la revendication 6, caractérisé en ce que les moyens (3, 4) d'élimination de particules parasites comprennent des moyens (3) de déviation des particules chargées (P) non absorbées par la cible (2) pour écarter celles-ci des neutrons produits en avant, de façon que ces derniers soient principalement des neutrons (N) d'énergie au moins égale à 15 MeV.

8. Générateur de neutrons selon l'une quelconque des revendications 1 à 7, caractérisé en ce que qu'il comprend en outre un collimateur (4) destiné à définir un champ d'irradiation.

9. Générateur de neutrons selon la revendication 7, caractérisé en ce qu'il comprend en outre un collimateur (4) destiné à définir un champ d'irradiation et en ce que lesdits moyens (3) de déviation sont prévus pour envoyer lesdites particules chargées (P) non absorbées par la cible (2) vers une paroi (4a) du collimateur (4).

10. Générateur de neutrons selon la revendication 9, caractérisé en ce qu'un bloc d'arrêt (12) muni d'un système réfrigérant (14) est placé dans ladite paroi (4a) du collimateur (4), pour arrêter lesdites particules chargées (P) non absorbées par la cible (2).

11. Générateur de neutrons selon la revendication 10, caractérisé en ce que ledit bloc d'arrêt (12) est essentiellement constitué de carbone ou d'hélium.

12. Générateur de neutrons selon l'une quelconque des revendications 7, 9, 10 et 11, caractérisé en ce que lesdits moyens (3) de déviation sont aptes à engendrer un champ magnétique pour provoquer ladite déviation.

13. Générateur de neutrons selon la revendication 12, caractérisé en ce que lesdits moyens (3) de déviation consistent en un aimant permanent.

14. Générateur de neutrons selon l'une quelconque des revendications 9 à 11, caractérisé en ce que les moyens (3) de déviation consistent en un aimant permanent au moins en partie intégré aux parois (4a, 4b, 4c, 4d) du collimateur (4), celles-ci étant aptes à constituer un circuit magnétique.

**Claims**

1. Generator for fast neutrons having a low content of low-energy neutrons, of the type comprising :

a source (1) of charged particles having an energy of at least 15 MeV,

a target (2) formed of a material adapted to emit neutrons under the impact of said charges particles, and

cooling means (8) for said target (2), characterized in that the target (2) comprises at least two elements (7) separate from one another and formed from lithium deuteride, the thickness of each of the elements being adapted such that the target mainly produces, forwardly directed neutrons having an energy of at least 15 MeV, and in that the cooling means (8) for the target (2) comprise circulation means enabling a gas which does not react chemically with lithium deuteride to be circulated between said elements.

2. Neutron generator according to Claim 1, characterized in that the charged particles are protons.

3. Neutron generator according to either of Claims 1 and 2, characterized in that the elements are formed from lithium 7 deuteride or natural lithium deuteride.

4. Neutron generator according to any one of Claims 1 to 3, characterized in that the target (2) has a thickness adapted according to a function of the mean energy of the charged particles which the source (1) is adapted to emit, and of the energy of the neutrons adapted to be produced, and comprises about M elements (7) consisting of discs, each disc having a different thickness, determined such that the charged particles emitted from source (1) lose therein 1/2 M of their energy, said discs (7) being aligned and spaced closer together as their distance from the source (1) of charged particles is increased.

5. Neutron generator according to any one of Claims 1 to 4, characterized in that said gas is helium.

6. Neutron generator according to any one of Claims 1 to 5, characterized in that it additionally comprises means (3, 4) for eliminating parasitic particles leaving the target (2) in a forwards direction.

7. Neutron generator according to Claim 6, characterized in that the elimination means (3, 4) for parasitic particles comprise means (3) for deviating charged particle (P) not absorbed by the target (2), to separate them from the forwardly directed neutrons, whereby the latter are mainly neutrons (N) having an energy of at least 15 MeV.

8. Neutron generator according to any one of Claims 1 to 7, characterized in that it additionally comprises a collimator (4) adapted to provide a radiation field.

9. Neutron generator according to Claim 7, characterized in that it additionally comprises a collimator (4) adapted to define a radiation field and in that said deviation means (3) are provided to direct said charges particles (P) not absorbed by the target (2) towards a wall (4a) of the collimator (4).

10. Neutron generator according to Claim 9, characterized in that an absorber block (12) having a cooling system (14) is located within said wall (4a) of the collimator (4), to halt said charged particles (P) not absorbed by the target (2).

11. Neutron generator according to Claim 10, characterized in that said absorber block (12) consists essentially of carbon or helium.

12. Neutron generator according to any one of Claims 7, 9, 10 and 11, characterized in that said deviation means (3) are adapted to provide a magnetic field to produce said deviation.

13. Neutron generator according to Claim 12, characterized in that said deviation means (3) comprise a permanent magnet.

14. Neutron generator according to any one of Claims 9 to 11, characterized in that the deviation means (3) consist of a permanent magnet, at least partially integral with the walls (4a, 4b, 4c, 4d) of the collimator (4) which are adapted to constitute a magnetic loop.

**Patentansprüche**

1. Neutronengenerator für hohe Energie mit einem geringen Anteil an Neutronen niederer Energie mit :

eine Quelle (1) für geladene Teilchen mit einer Energie von wenigstens gleich 15 MeV,

einem Target (2) aus einem Material, welches beim Auftreffen dieser geladenen Teilchen Neutronen aussendet, und

einer Kühleinrichtung (8) für dieses Target (2), dadurch gekennzeichnet, daß das Target (2) von wenigstens zwei voneinander getrennten Elementen (7) gebildet ist, die aus Lithiumdeuterid bestehen, wobei die Dicke eines jeden dieser Elemente angepaßt ist, damit das Target im wesentlichen in Vorwärtsrichtung Neutronen mit einer Energie von wenigstens gleich 15 MeV erzeugt, und daß die Kühleinrichtung (8) für das Target (2) aus Strömungsmitteln besteht, die ermöglichen, ein chemisch nicht mit dem Lithiumdeuterid reagierendes Gas zwischen den Elementen durchströmen zu lassen.

2. Neutronengenerator nach Anspruch 1, dadurch gekennzeichnet, daß die geladenen Teilchen Protonen sind.

3. Neutronengenerator nach Anspruch 1 oder 2, dadurch gekennzeichnet, daß die Elemente aus Deuteron-Lithium 7 oder aus natürlichem Lithiumdeuterid sind.

4. Neutronengenerator nach irgendeinem der Ansprüche 1 bis 3, dadurch gekennzeichnet, daß das Target (2) eine Dicke aufweist, die als Funktion der mittleren Energie der geladenen Teilchen, welche die Quelle (1) aussenden kann, und der Energie der zu erzeugenden Neutronen eingestellt ist, und ungefähr M Elemente (7) umfaßt, die aus Tabletten bestehen, wobei jede Tablette eine unterschiedliche Dicke aufweist, die so berechnet ist, daß die von der Quelle (1) ausgehenden, geladenen Teilchen dort den Anteil 1/2 M ihrer Energie verlieren und daß diese Tabletten (7) beabstandet und mit von der Quelle (1) für die geladenen Teilchen her abnehmender Dicke angeordnet sind.

5. Neutronengenerator nach irgendeinem der Ansprüche 1 bis 4, dadurch gekennzeichnet, daß das Gas Helium ist.

6. Neutronengenerator nach irgendeinem der Ansprüche 1 bis 5, dadurch gekennzeichnet, daß er ferner Mittel (3, 4) zum Entfernen von Untergrundteilchen umfaßt, die in Vorwärtsrichtung von dem Target (2) ausgehen.

7. Neutronengenerator nach Anspruch 6, dadurch gekennzeichnet, daß die Mittel (3, 4) zum Entfernen von Untergrundteilchen Ablenkmittel (3) für die von dem Target (2) nicht absorbierten, geladenen Teilchen umfaßt, um diese von den in Vorwärtsrichtung erzeugten Neutronen zu entfernen, derart, daß letztere hauptsächlich Neutronen (N) mit einer Energie von wenigstens gleich 15 MeV sind.

8. Neutronengenerator nach irgendeinem der Ansprüche 1 bis 7, dadurch gekennzeichnet, daß er ferner einen Kollimator (4) umfaßt, mit dem ein Bestrahlungsfeld begrenzbar ist.

9. Neutronengenerator nach Anspruch 7, dadurch gekennzeichnet, daß er ferner einen Kollimator (4) umfaßt, um ein Bestrahlungsfeld zu begrenzen, und daß die Ablenkmittel (3) vorgesehen sind, um die von dem Target (2) nicht absorbierten, geladenen Teilchen (B) zu einer Wand (4a) des Kollimators (4) zu lenken.

10. Neutronengenerator nach Anspruch 9, dadurch gekennzeichnet, daß ein mit einem Kühlsystem (14) versehener Auffangblock (12) in der genannten Wand (4a) des Kollimators (4) angeordnet ist, um die von dem Target (2) nicht absorbierten, geladenen Teilchen (B) anzuhalten.

11. Neutronengenerator nach Anspruch 10, dadurch gekennzeichnet, daß der Auffangblock (12) im wesentlichen aus Kohlenstoff oder Helium besteht.

12. Neutronengenerator nach irgendeinem der Ansprüche 7, 9, 10 oder 11, dadurch gekennzeichnet, daß mit den Ablenkmitteln (3) ein Magnetfeld erzeugbar ist, um die Ablenkung hervorzurufen.

13. Neutronengenerator nach Anspruch 12, dadurch gekennzeichnet, daß die Ablenkmittel (3) aus einem Permanentmagnet bestehen.

14. Neutronengenerator nach irgendeinem der Ansprüche 9 bis 11, dadurch gekennzeichnet, daß die Ablenkmittel (3) aus einem Permanentmagnet bestehen, der wenigstens teilweise in die Wände (4a, 4b, 4c, 4d) des Kollimators (4) integriert ist, wobei durch diese ein magnetischer Kreis gebildet werden kann.